# EUROPEAN PATENT SPECIFICATION

(11) **EP 3 213 778 B1**
(45) Date of publication and mention of the grant of the patent: **15.03.2023**
(21) Application number: 17150192.7
(22) Date of filing: 29.09.2003
(51) Int. Cl.: A61P 17/00, A61P 19/00, A61L 27/38, A61K 35/12, C12N 5/00

(54) **CELLS ON A SUPPORT MATRIX FOR TISSUE REPAIR**
ZELLEN AUF MATRIXUNTERLAGEN FÜR DIE WIEDERHERSTELLUNG VON GEWEBEN
CELLULES SEMÉES SUR UNE MATRICE DE SUPPORT POUR EFFECTUER DES RÉPARATIONS TISSULAIRES

(30) Priority: 27.09.2002 US 414098 P
(43) Date of publication of application: 06.09.2017
(62) Divisional of application: 03750654.0
(73) Proprietor: Vericel Corporation, Ann Arbor, MI 48105-9755 (US)
(72) Inventor: MING, Hao Zheng, City Beach, Western Australia 6015 (AU); GIANNETTI, Bruno, 53347 Bonn (DE)
(74) Representative: Carpmaels & Ransford LLP

(56) References cited:
- WO-A1-01/08610
- US-A- 5 306 311
- US-A- 6 120 514
- BONASSAR LAWRENCE J ET AL: "Tissue engineering: The first decade and beyond", JOURNAL OF CELLULAR BIOCHEMISTRY SUPPLEMENT, vol. 0, no. 30-31, 1998, pages 297-303, XP009032638, ISSN: 0733-1959

## Description

Some type of tissue defect occurs in every single person in one aspect or another. Burns, scrapes, muscle, cartilage, or tendon tears, nerve damage, broken bones, and the like are commonplace among people with active lifestyles.

Using cartilage repair as a typical example, more than 500,000 arthroplastic procedures and total joint replacements are performed each year in the United States. Approximately the same number of similar procedures are performed in Europe. Included in these numbers are about 90,000 total knee replacements and around 50,000 procedures to repair defects in the knee per year (In: Praemer A., Furner S., Rice, D. P., Musculoskeletal conditions in the United States, Park Ridge, Ill.: American Academy of Orthopaedic Surgeons, 1992, 125).

U.S. Patent Nos. 5,759,190; 5,989,269; 6,120,514; 6,283,980; 6,379,367; 6,569,172; 6,592,598; 6,592,599; and 6,599,300 describe various embodiments of methods and compositions for treating cartilage defects by implanting a component seeded with chondrocytes at the site of a cartilage defect.

WO 01/08610 concerns a kit and a method for the treatment of articulating joint surface cartilage by the transplantation of chondrocytes to a surface to be treated. US 5306311 concerns a prosthetic, resorbable articular cartilage and methods of its fabrication and insertion. Bonassar & Vacanti, "Tissue engineering: The first decade and beyond", J Cell Biochem. 1998; 72 Suppl 30-31 (S30-31): 297-303, reviews developments in the field of tissue engineering. US 6120514 concerns methods for effective chondrocyte and/or cartilage transplantation to articulating joint surfaces, and a kit providing materials for the practice of the method.

Currently, there is a need for efficient and effective methods for repairing and/or regenerating defective tissues other than cartilage. The teachings of the instant invention provide for effective and efficient means of promoting the repair and regeneration of defective tissues using cell-seeded support matrices.

The present invention is as defined in the appended claims. The present invention relates to compositions for use in methods for the effective treatment of tissue defects and for tissue regeneration using cells, preferably autologous cells, seeded on a support matrix. The present invention also relates to tissue repair compositions comprising a membrane seeded with cells of one or more specific types for use in repairing and/or regenerating one or more specific tissues.

In one embodiment, the present disclosure relates to a series of methods and products for the effective treatment of any type of tissue defect, including but not limited to muscle, soft tissue, bone, tendon, nerve, and cartilage tissue, or for tissue regeneration, by the transplantation of cells (e.g., autologous) seeded on a support matrix. In some embodiments of the disclosure, the methods may also include use of non-autologous stem cells, a covering patch and/or a hemostatic barrier. In one embodiment, the covering patch and/or hemostatic barrier can be any matrix material or adhesive described herein. A detailed description of autologous transplantation and several support matrices, covering patches, and/or hemostatic barriers are described in U.S. Patent No. 6,379,367, issued April 30, 2002.

### A. Cells and Tissues

The present invention comprises the use of compositions that include cells, preferably autologous cells, seeded onto a support matrix for use in tissue repair and/or regeneration. By "seeding" is meant that cells are brought into contact with a support matrix, and adhere (with or without an adhesive) to the support matrix for a period of time prior to transplantation. In one embodiment, cells adhere to and proliferate and differentiate into a desired cell type on the support matrix prior to transplantation.

In an embodiment of the invention, the cells are retained only on one surface or an edge of, or to a specified depth (as described herein) of the support matrix, i.e., the cells are adhered to one surface or are adjacent the support matrix, such as described in U.S. Publication No. 20020173806.

In the present invention, uniform seeding is preferable. It is believed that the number of cells seeded does not limit the final tissue produced, however optimal seeding may increase the rate of generation. Optimal seeding amounts will depend on the specific culture conditions. In one embodiment, the matrix is seeded with from about 0.05 to about 5 times the physiological cell density of a native tissue type, i.e., in nerve or tendon. In another embodiment, the cell density can be less than about 1×10⁵ to 1×10⁸ cells, or more, per ml., typically about 1×10⁶ cells per ml.

By way of example and not by limitation, suitable cells for use in the disclosure include tenocytes, myocytes, stem cells, osteocytes, chondrocytes, epithelial cells, keratinocytes, nerve cells (including, but not limited to neurocytes, astrocytes, dendritic cells, and glial cells), fibroblasts, odontocytes, synoviocytes, adipocytes, and cementocytes. In addition, precursor cells to these cell types are also useful in the present disclosure. In one embodiment, for example, myoblasts, which are precursors to myocytes; osteoblasts, which are precursors to osteocytes; and neuroblasts, which are precursors to neurocytes, are all useful in the present disclosure. In one embodiment, preferably the cells and cell precursors are autologous cells and autologous cell precursors.

Tissues that would benefit from the methods and compositions of the present disclosure include, but are not limited to, tendons, muscles, cartilage, bone and teeth, skin, neural tissue, epithelial tissue, and other tissues.

### B. Methods

In one aspect of the present invention, the present invention provides the use of compositions comprising autologous cells to treat many different tissue defects and to regenerate tissue.

By way of example, and not by limitation, the present disclosure provides a method for treating tendon tears by transplanting autologous tenocytes onto a support matrix. One representative example of a tendon tear is rotator cuff tendonitis, caused by a partial tendon tear. The present disclosure also includes methods for cultivation of tenocytes, seeding of tenocytes on a support matrix, and implantation of the tenocyte-seeded support matrix into or over the site of transplantation.

The present disclosure also contemplates use of the methods taught herein to treat bone defects and to regenerate bone. In one embodiment, autologous osteoblasts are seeded onto a support matrix and the cell-seeded support matrix is implanted into or over the site of transplantation. Such representative examples of bone defects include non-union fractures, bone segmental defect or reconstructive surgery using bone tissue. The present disclosure also provides a method for the cultivation of osteoblasts, seeding of osteoblasts onto a support matrix, and implantation of the cell-seeded support matrix into or over the site of transplantation.

The present disclosure also contemplates use of the methods taught herein to treat muscle defects and to regenerate muscle. In one embodiment, autologous myoblasts are seeded onto a support matrix and the cell-seeded support matrix is implanted into or over the site of transplantation. Representative examples of a muscle defect includes muscle degeneration and muscle tears. The present disclosure also provides a method for the cultivation of myoblasts, seeding of myoblasts onto a support matrix, and implantation of the cell-seeded support matrix into or over the site of transplantation.

The present invention comprises the compositions for use of the invention to treat cartilage defects and to regenerate cartilage. In one embodiment, autologous chondrocytes are seeded onto a support matrix and the cell-seeded support matrix is implanted into or over the site of transplantation. One representative example of a cartilage defect includes deterioration or injury of the cartilage in a joint, such as the knee, shoulder, elbow, hip, or ankle. The present invention comprises seeding of chondrocytes onto a support matrix, and implantation of the cell-seeded support matrix into or over the site of transplantation.

The present disclosure also contemplates use of the methods taught herein to treat skin defects and to regenerate skin. In one embodiment, autologous keratinocytes are seeded onto a support matrix and the cell-seeded support matrix is implanted into or over the site of transplantation. Some representative examples of skin defects include partial- and full-thickness wounds due to burn, chronic non-healing, venous stasis, and diabetic ulcers. The present disclosure also provides a method for the cultivation of keratinocytes, seeding of keratinocytes onto a support matrix, and implantation of the cell-seeded support matrix into or over the site of transplantation.

The present disclosure also contemplates use of the methods taught herein to treat urinary tract defects and diseases (e.g., incontinence), and to regenerate epithelial tissue. In one embodiment, autologous epithelial cells are seeded onto a support matrix and the cell-seeded support matrix is implanted into or over the site of transplantation in the urinary tract. The present disclosure also provides a method for the cultivation of epithelial cells, seeding of epithelial cells onto a support matrix, and implantation of the cell-seeded support matrix into or over the site of transplantation.

The present disclosure also contemplates use of the methods taught herein to treat nerve defects and to regenerate nerves. In one embodiment, autologous nerve cells are seeded onto a support matrix and the cell-seeded support matrix is implanted into or over the site of transplantation. One representative example of a nerve defect includes spinal cord injury or nerve damage caused by burns. The present disclosure also provides a method for the cultivation of nerve cells, seeding of nerve cells onto a support matrix, and implantation of the cell-seeded support matrix into or over the site of transplantation.

The present disclosure also contemplates a method for increasing the amount of adipose tissue in a patient. By way of example, increased adipose tissue may be desired during plastic or reconstructive surgery, such as, breast augmentation or reconstruction.

The present disclosure also contemplates use of the methods taught herein to produce adipocytes for use in plastic or reconstructive surgery (e.g., breast augmentation or reconstructive surgery). In one embodiment, autologous adipocytes are seeded onto a support matrix and the cell-seeded support matrix is implanted into or over the site of transplantation. The present disclosure also provides a method for the cultivation of adipocytes, seeding of adipocytes onto a support matrix, and implantation of the cell-seeded support matrix into or over the site of transplantation.

The present disclosure also contemplates use of the methods taught herein to treat any tissue defect or to regenerate any tissue. In one embodiment, autologous stem cells are differentiated, partially differentiated, or undifferentiated prior to seeding on the support matrix, and then are seeded onto a support matrix and the cell-seeded support matrix is implanted into or over the site of transplantation. Optionally, factors to assist in differentiation may be used before, during, or after transplantation of the cell-seeded support matrix. The present disclosure also provides method for the cultivation and differentiation of the stem cells, seeding of the stem cells or differentiated cells onto a support matrix, and implantation of the cell-seeded support matrix into or over the site of transplantation.

### C. Compositions

Cells are brought into contact with one or more predetermined portions of a support matrix, for example with one surface or portion of a surface of a support matrix, such that a substantial portion of the cells or substantially all of the cells migrate into one or more of the surfaces of the support matrix up to a predetermined maximum depth of the support matrix. That depth is up to about 50 percent, preferably up to 25 percent, more preferably up to about 10 percent and even more preferably up to about 3-5 percent of the depth of the support matrix. Such controlled seeding of the cells on and/or near a surface of the support matrix allows cells to freely migrate and populate a site of transplantation and leads to enhanced proliferation of the cells and regeneration of tissue at the transplantation site. In one embodiment, such seeding can be accomplished with or without vacuum by pouring the cells on or near a surface of the support matrix, such as described in U.S. Publication No. 20030134411 or mixing or placing cells into a portion of the support matrix. The cells can be obtained in any suitable manner, including but not limited to cells obtained from a biopsy. The cells thus obtained can then be isolated, cultured and seeded onto a support matrix, forming a composition for use according to the present disclosure, as described below.

### 1. Obtaining Cells

Cells can be isolated from tissue in a variety of ways, all which are known to one skilled in the art. In one embodiment, cells can be isolated from a biopsy material by conventional methods. The biopsy material can be extracted from any tissue of the patient relating to the tissue type of the defect or tissue regeneration. For example, a patient requiring treatment or regeneration of a tendon can have a biopsy taken from any tendon in the body. Such tendons include, but are not limited to tendon of flexor carpi radialis and the calcaneus tendon. From the tendon biopsy, tenocytes are isolated and cultured by conventional methods.

Likewise, a patient requiring treatment of rotator cuff tendonitis can have a biopsy taken from any tendon. Such tendons include, but are not limited to flexor carpi radialis and the calcaneus tendon. From the tendon biopsy, osteoblasts are isolated and cultured by conventional methods.

For treatment of soft tissue defects, such as a skin defect (for example, a burn, gash, or laceration), a skin biopsy may be taken from any portion of the epidermis of the patient containing keratinocytes. From the skin biopsy, keratinocytes are isolated and cultured by conventional methods.

For other soft tissue defects, such as defects in epithelial lining of the bladder, a biopsy may be taken from urethral tract, from which epithelial cells may be isolated. Epithelial cells may be isolated from tissues including, but are not limited to fossa navicularis urethrae. From the urethral biopsy, epithelial cells are isolated and cultured by convention methods.

For treatment of bone defects, a biopsy can be taken from any bone in the body. Such bones include, but are not limited to the iliac crest. From the bone biopsy, osteoblasts are isolated and cultured by conventional methods.

For treatment of a cartilage defect, a cartilage biopsy may be taken from any type of cartilage in the body, including, but not limited to articular cartilage and meniscal cartilage, depending on the type of cartilage the site of the defect or to be regenerated. In the case of cartilage, the type of cartilage is not relevant to the method for treating the defect. Thus, cells in an articular cartilage biopsy may be used to treat a meniscal cartilage defect and vice versa. Meniscal cartilage can be obtained from, for example, the knee. Articular cartilage is a more specialized type of hyaline cartilage and can be found in any joint surface. Chondrocytes obtained from any articular surface can be used for the treatment of any cartilage defect. Such materials include, but are not limited to the knee joint.

For treatment of a nerve defect, a nerve cell biopsy may be taken from any peripheral never or spinal cord. From the biopsy, nerve cells are isolated and cultured by conventional methods.

Alternatively, to treat any type of tissue defect, a biopsy containing stem cells may be taken from bone marrow, umbilical cord blood, skin, or cartilage of a patient. From the biopsy, stem cells from the patient are isolated and cultured. The stem cells are differentiated into the cells specific for use in treatment of the specific tissue defect.

Stem cells are also isolated from fetal tissue and umbilical cord by conventional methods. Stem cells may be autologous or non-autologous as certain stem cells are only available in umbilical cord blood, but can differentiate into a required cell type. Any type of stem cells, including hematopoietic stem cells, mesenchymal stem cells, totipotent stem cells, and pluripotent stem cells, can be used in the present disclosure, depending on the particular defect to be repaired or tissue to be regenerated.

### 2. Incubation, Isolation and Culturing of Cells

Once the biopsy is extracted, the biopsy is washed and incubated in a cell growth medium containing an appropriate enzyme that will dissolve the biopsy material surrounding the cells within the tissue without harming the cells, for a prescribed period of time. The cell growth medium is specific for the type of cell being extracted from the biopsy. In one embodiment of the invention, the cell growth medium includes 20% fetal calf serum, and optionally an antibiotic, an antifungal, and factor(s) necessary for the induction of lineage cell differentiation (hereinafter "cell growth medium"). For example, one factor necessary for chondrocyte differentiation in culture from a primary chondrocyte culture isolated from a cartilage biopsy is ascorbic acid. Another factor necessary for chondrocyte differentiation from stem cells in culture is transforming growth factor-beta.

In one embodiment, the enzyme included in the cell growth medium is preferably a trypsin/EDTA solution. Alternatively, the enzyme can be collagenase.

In one embodiment, after incubation, the biopsy material is washed again, and weighed. In order to obtain an adequate number of cells to start a cell culture, the biopsy material weighs between 80 and 300 milligrams. Preferably, the biopsy material weighs at least between 200 and 300 milligrams.

In one embodiment, the biopsy material is then digested, preferably with a digestive enzyme that will not harm the cells, by incubating the biopsy material in a solution of the digestive enzyme and cell culture medium for about 5 to about 30 hours, preferably, about 15 to about 20 hours at 37 degrees Celsius in a 5% CO₂ atmosphere. The digestive enzyme can be for example, crude collagenase, for digestion of any type of collagen. In one embodiment, the biopsy material preferably is minced to aid in digestion of the material.

In one embodiment, after digestion, the cells from the biopsy material are isolated by centrifuging the biopsy solution, and washing the resulting pellet with cell growth medium. Alternatively, the minced material may first be strained through a mesh having a pore size appropriate for the particular cell type to remove larger debris and isolate the cells. The isolated cells are then counted and assessed for viability.

In one embodiment, following isolation, the cells are cultured in cell growth medium for about 3 days to about five weeks, at 37 degrees Celsius in a 5% CO₂ atmosphere. The time period for cell culturing can vary with cell type. Culturing time may vary with different cell types since different cell types have different rates of proliferation.

### 3. Support Matrices

Once the cells have been cultured to an adequate density, the cells are then seeded onto a support matrix.

The support matrix can be in any form suitable for cell adherence with or without an adhesive. By way of example and not limitation, the support matrix can be in the form of a membrane, microbeads, fleece, threads, or a gel, and/or mixtures thereof. The support matrix material can have other physical or mechanical attributes, such as acting as a hemostatic barrier. A hemostatic barrier inhibits penetration of adjunct cells and tissue into the treated defect area.

The support matrix is a semi-permeable material which includes cross-linked or uncross-linked collagen, preferably type I in combination with type III, or type II. The support matrix may also include polypeptides or proteins obtained from natural sources or by synthesis, such as hyaluronic acid, small intestine submucosa (SIS), peritoneum, pericardium, polylactic acids and related acids, blood (i.e., which is a circulating tissue including a fluid portion (plasma) with suspended formed elements (red blood cells, white blood cells, platelets), or other material which is bioresorbable. Bioabsorbable polymers, such as elastin, fibrin, laminin and fibronectin are also useful in the present disclosure. Support matrix materials as described in U.S. Publication No. 20020173806 are also useful in the present disclosure.

In addition, the support matrix preferably is initially (i.e., before contact with the cells to be transplanted) free of intact cells and is resorbable within the patient. The support matrix may have one or several surfaces, such as a porous surface, a dense surface, or a combination of both. The support matrix may also include semi-permeable, impermeable, or fully permeable surfaces. Support matrices having a porous surface are described, for example, in U.S. Patent No. 6,569,172.

The support matrix is autologous or allogeneic. In one embodiment, a suitable autologous support matrix is formed from blood, as exemplified in U.S. Patent No. 6,368,298, issued to Berretta, et al. on Apr. 9, 2002.

A suitable support matrix will be a solid, semi-solid, gel, or gel-like scaffold characterized by being able to hold a stable form for a period of time to enable the adherence and/or growth of cells thereon, both before transplant and after transplant, and to provide a system similar to the natural environment of the cells to optimize cell growth and differentiation. Examples of suitable support matrices are disclosed in U.S. Publication No. 20020173806. In one embodiment, the support matrix and/or cells, either individually or in combination, may be combined with an adhesive (e.g., a biocompatible glue such as fibrin glue which may be autologous or allogeneic) or physical or mechanical retention means such a resorbable pin to assist in retaining the repair structures according to the present invention in or over the site of transplantation. Additional examples of support matrices include those described in U.S. Patent Application No. 10/427,463, filed May 1, 2003.

The support matrix can be cut or formed into any regular or irregular shape. In a preferred embodiment, the support matrix can be cut to correspond to the shape of the defect. The support matrix can be flat, round and/or cylindrical in shape. The shape of the support matrix can also be molded to fit the shape of a particular tissue defect. If the support matrix is a fibrous material, or has the characteristics of a fiber, the support matrix can be woven into a desired shape. Alternatively, the support matrix can be a gel, gel-like, or non-woven material.

In one embodiment, a support matrix can be seeded with multiple cell types and have different cell types on and/or in and/or throughout and/or adjacent to different portions of the support matrix. By way of example, one portion of the support matrix may include a first cell type (e.g., tendon cells) and another portion of the matrix may include a second cell type (e.g., muscle cells). For example, to repair a bone and cartilage defect at the intersection of bone and cartilage, one portion of the support matrix may include chondrocytes and another portion of the matrix may include osteocytes.

By way of further example, if the matrix is disc shaped, having two sides and an edge, a first side can include a first cell type (e.g., tendon cells) thereon and the second side or edge can include a second cell type (e.g., muscle cells) thereon. Alternatively, each surface of a support matrix can include the same cell type in and/or on and/or throughout and/or adjacent to a surface. Preferably, the cells are seeded in such a way that the cells are prevented from migrating from one side to the other. Thus, in some embodiments, the cell types will not interact with each other.

In another embodiment, two or more support matrices can be in contact with each other. In such an embodiment, a first support matrix can be in contact with a second support matrix either before, during or after either support matrix is contacted with one or more cell types.

### D. Implantation of the Composition

After the cells are seeded onto the support matrix, the support matrix and the cells are transplanted into the tissue defect, with cells facing the surface to be treated. In one embodiment, a covering patch is secured (e.g., biocompatible adhesive or suture) over the defect as described herein, and the defect is permitted to heal on its own.

In one embodiment, a covering patch serves to cover the defect to further prevent infiltration of undesired materials, such as fibroblasts or macrophages, from the surrounding environment. In one embodiment, the covering patch may be any of the support matrices described herein, and/or can include collagen (type I/III), hyaluronic acid, fibrin and polylactic acid. Preferably, the covering patch is cell-free and resorbable, and may be semi-permeable.

In one embodiment, the support matrix and cells are injectable to the site of transplantation, with or without an adhesive or glue.

### E. Other Materials

A support matrix or seeded support matrix can also include various pharmacological actives including but not limited to antimicrobials, antivirals, antibiotics, growth factors suitable to the type of tissue to be regenerated and/or repaired, blood clotting modulators such as heparin and the like, as well as mixtures and composite layers thereof can be added to the biocompatible biodegradable support matrix material, prior to impregnation into the support matrix.

A support matrix or seeded support matrix can also include growth factors such as autologous and non-autologous growth factors suitable to the type of tissue to be regenerated and/or repaired, including but not limited to transforming growth factor (such as TGF-beta-3), bone morphogenetic protein (such as BMP-2), PTHrP, osteoprotegrin (OPG), Indian Hedgehog, RANKL, and insulin-like growth factor (IgF1), as described in U.S. Publication No. 20030144197.

As noted above, the present invention can also include a biocompatible glue in contact with a substrate and/or biodegradable material and/or cells. Such biocompatible glues or adhesives can include an organic fibrin glue (e.g., Tisseel^{®}, fibrin based adhesive available from Baxter, Austria, or a fibrin glue prepared in the surgical theater using autologous blood samples). In one embodiment, cells can be mixed with an appropriate glue before, during and/or after contact with a support matrix. Alternatively, an appropriate glue can be placed in a defect or layered on top of cells or as a layer below cells on a surface or edge or impregnated in a support matrix.

In one embodiment, the present invention includes cells and glue combined together in a mixture of glue and cells or one or more alternating layers of cells and glue on a surface or edge of a support matrix. It is contemplated that cells that are autologous can be transplanted into a defect. Cells are mixed, either homogeneously or non-homogeneously, with a suitable glue before application of the cell/glue mixture to a support matrix. Preferably, the glue and the cells are mixed immediately (that is, in the operating theater) before applying the glue and cells to the support matrix and implantation of the combination of glue, cells and support matrix to a defect. Alternatively cells and a glue are alternately applied in one or more layers to a support matrix. In one embodiment, a glue for use in the present invention is a bio-compatible glue, such as a fibrin glue, and more specifically either an autologous fibrin glue or a non-autologous fibrin glue. Preferably, an autologous fibrin glue is used.

The following examples describe methods suitable for practicing several embodiments of the present disclosure.

### F. EXAMPLES

### Example 1: Method of Treating Tendonitis

A biopsy is taken from the tendon of flexor carpi radialis or calcaneus tendon, and washed in DMEM, then cleaned of adipose tissue. The tissue is minced and digested in 0.25% trypsin in serum-free DMEM for 1 hour at 37 degrees Celsius, followed by a 5 hour digestion in 1 milligram per milliliter collagenase in serum-free Dulbecco's Modified Essential Medium (DMEM) at 37 degrees Celsius. The cell pellet is washed 2 to 3 times (centrifuged at 200 g for about 10 minutes), and resuspended in growth medium (DMEM containing 10% fetal calf serum (FCS), 50 micrograms per milliliter ascorbic acid, 70 micromole/liter gentamycin sulfate, 2.2 micromole/liter amphotericin). The tenocytes are counted to determine viability and then seeded. The culture is maintained in a humidified atmosphere of 5% CO₂, 95% air in a CO₂ incubator at 37 degrees Celsius and handled in a Class 100 laboratory. The medium is changed every 2 to 3 days. Other compositions of culture medium may be used for culturing the cells. The cells are then trypsinized using trypsin EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH, (Kaiserstr., Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally is covered with a covering patch. The defect is then permitted to heal on its own.

### Example 2: Method of Treating Bone Defects

A biopsy is taken from the iliac crest, and cut into small pieces before placing into a tissue culture flask. The cells that migrated from the bone pieces were dispersed by collagenase digestion. The osteoblasts are isolated and counted to determine viability. The osteoblasts are maintained in monolayer culture with alpha-MEM containing 10% fetal bovine serum (FBS), 2 millimolar of beta-glycerophosphate and 50 micrograms per milliliter of L-ascorbic acid. The culture is maintained in a humidified atmosphere of 5% CO₂, 95% air at 37 degrees Celsius in a CO₂ incubator at 37 degrees Celsius and handled in a Class 100 laboratory. The medium is changed every 2-3 days. Other compositions of culture medium may be used for culturing the cells. The cells are trypsinized using trypsin and EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH, (Kaiserstr., Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally is covered with a covering patch. The defect is then permitted to heal on its own.

### Example 3: Method of Treating Muscle Defects

A biopsy is taken from M. gastrocnemius muscle. The biopsy is washed in Ham's F12 supplemented with 10 millimolar Hepes/NaOH (pH 7.2), and cleaned of tendons and fat tissue. The tissue is cut into small pieces, then incubated in the dissociation buffer, which is the above buffer containing 0.12% (w/v) pronase and 0.03% (w/v) EDTA, for 1 hour at 37 degrees Celsius in a shaking water bath. After digestion, the suspension is filtered through a 100 micrometer nylon mesh into an equal volume of the culture medium which is Ham's F12 containing 2.2 grams per liter of natrium bicarbonate, 20% fetal calf serum (FCS) and penicillin and streptomycin. The cell pellet is washed by centrifuging at 300 g for 10 minutes at 4 degrees Celsius and the pellet is resuspended in the culture medium. The muscle cells are isolated and counted to determine viability. The myoblasts are cultured and maintained in a humidified atmosphere of 5% CO₂, 95% air in a CO₂ incubator at 37 degrees Celsius and handled in a Class 100 laboratory. The medium is changed 24 hour after seeding and then every 4 days. Other compositions of culture medium may be used for culturing the cells. The cells are trypsinized using trypsin EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH, (Kaiserstr., Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally is covered with a covering patch. The defect is then permitted to heal on its own.

### Example 4: Method of Treating Cartilage Defects

A biopsy is taken from the knee and the biopsy is washed once in cell growth medium. The growth medium contains 70 micromole/liter gentomycin sulfate, 2.2 micromole/liter amphotericin, 0.3 millimole/liter ascorbic acid, and 20% fetal calf serum. The biopsy is incubated in cell growth medium containing trypsin EDTA for 5 to 10 minutes at 37 degrees Celsius and at 5% CO₂. The biopsy is washed two or three more times with cell culture medium to remove any remaining trypsin EDTA. The biopsy is weighed, and then digested with collagenase (about 5000 units for an 80-300 milligram biopsy) for about 3 to 12 hours at 37 degrees Celsius and at 5% CO₂. Alternatively, the biopsy is minced at this point to aid in digestion of the material. The biopsy material is then centrifuged at 700 g for about 10 minutes, and the pellet is washed with cell growth medium. The chondrocytes are isolated and counted to determine viability. The chondrocytes are cultured.

Chondrocytes are grown in minimal essential culture medium containing HAM F12 and 15 millimolar Hepes buffer and 5 to 10% autologous serum in a CO₂ incubator at 37 degrees Celsius and handled in a Class 100 laboratory. Other compositions of culture medium may be used for culturing the cells. The cells are trypsinized using trypsin EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH, (Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally covered with a covering patch. The defect is then permitted to heal on its own.

### Example 5: Method of Treating Skin Defects

A biopsy is taken from human skin. The biopsy is washed once in cell growth medium. The growth medium contains 70 micromole/liter gentomycin sulfate, 2.2 micromole/liter amphotericin, 0.3 millimole/liter ascorbic acid, and 20% fetal calf serum. The biopsy is incubated in cell growth medium containing trypsin EDTA for 5 to 10 minutes at 37 degrees Celsius and at 5% CO₂. The biopsy is washed two or three more times with cell culture medium to remove any remaining trypsin EDTA. The biopsy is weighed, and then digested with collagenase (about 5000 units for an 80-300 milligram biopsy) for about 17 to 21 hours at 37 degrees Celsius and at 5% CO₂. The biopsy may be minced at this point to aid in digestion of the material. The biopsy material is then centrifuged at 700 g for about 10 minutes, and the pellet is washed with cell growth medium. The keratinocytes are isolated and counted to determine viability. The keratinocytes are cultured.

The keratinocytes are cultivated in the presence of NIH 3T3 fibroblasts in DMEM/F12 culture medium containing 10% fetal bovine serum, hydrocortisone (0.4 micrograms per milliliter), human epidermal growth factor (10 nanograms per milliliter) 10-10M cholera toxin and 5 micrograms per milliliter of zinc-free insulin, 24 micrograms per milliliter adenine, and 2×10⁹ molar 3,3,5-triiodo-L-thyronine.

A type I/III collagen membrane from Geistlich Sohne (Switzerland) or Matricel GmbH, (Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 4 days with a medium change at day 2.

At the end of the incubation period the medium is decanted and the cell-seeded support matrix is implanted, cell-side down, into the defect site, and optionally is covered with a covering patch. The defect is then permitted to heal on its own.

### Example 6: Method of Treating Epithelium Defects

A biopsy from the upper or lower urinary tract is collected and transported in calcium-free, magnesium-free HBSS (Hank's balance salt solution) with 0.35 grams per liter sodium bicarbonate containing 10 millimolar 4-(2-hydroxyethyl)-1-piperazine-ethanesulfonic acid (HEPES) buffer and 100 KJU per milliliter aprotinin. The specimen is washed twice in HBSS, and excess stromal tissue is removed aseptically. The tissue is then cut into 3 cubic millimeter pieces before digestion in 0.1% EDTA overnight at 4 degrees Celsius. The cell pellet is rinsed 2 to 3 times (centrifuged at 200 g for about 10 minutes) in the growth medium which is a low calcium serum-free medium formulated for primary keratinocyte culture. This medium is supplied with recombinant epidermal growth factor and bovine pituitary extract as additives. Cholera toxin is added to the medium at a final concentration of 30 nanograms per milliliter. The uroepithelial cells are isolated and counted to determine viability. The cells are seeded and maintained in a humidified atmosphere of 5% CO₂, 95% air in a CO₂ incubator at 37 degrees Celsius and handled in a Class 100 laboratory. The medium is changed 3 times a week. Other compositions of culture medium may be used for culturing the cells. The cells are trypsinized using trypsin EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH (Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). This particular support matrix is first pre-treated with either 0.6% glutaraldehyde for 1 minute or with Tisseel^{®} (Immuno AG, Vienna, Austria), which is a fibrin glue. These treatments delay the resorption of the matrix significantly. This support matrix is washed several times in distilled water until nonreacted glutaraldehyde is removed. A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally is covered with a covering patch. The defect is then permitted to heal on its own.

### Example 7: Method of Treating a Spinal Cord Defects

A biopsy is taken from any peripheral nerve or spinal cord. Human peripheral nerves are maintained in DMEM with 10% PBS, 100-micrograms per milliliter penicillin and 100 micrograms per milliliter streptomycin. The epineurium is removed and nerve fascicles are cut into 1 to 2 millimeter-long segments. Explants from the segments are maintained in the above medium to induce an in vitro Wallerian degeneration for 14 days. During this period the medium is changed every other day. After 14 days the explants are digested in 1300 units per milliliter collagenase and 10 units per milliliter dispase in DMEM with continuous agitation at 37 degrees Celsius for 1 hour, then the digested tissue is further dissociated by repeated trituration through a Pasteur pipette. The cell pellet is washed and resuspended in DMEM with 10% FBS before seeding on culture dishes that had been coated with type I rat tail collagen. Nerve cell cultures are maintained in a humidified atmosphere of 5% CO₂, 95% air in a CO₂ incubator at 37 degrees Celsius and handled in a Class 100 laboratory. Other compositions of culture medium may be used for culturing the cells. The cells are trypsinized using trypsin EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH (Kaiserstr., Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). This particular support matrix is first pre-treated with either 0.6% glutaraldehyde for 1 minute or with Tisseel^{®} (Immuno AG, Vienna, Austria), which is a fibrin glue. These treatments delay the resorption of the matrix significantly. This support matrix is washed several times in distilled water until nonreacted glutaraldehyde is removed. A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally is covered with a covering patch. The defect is then permitted to heal on its own.

### Example 8: Method of Treating any Tissue Defects

A biopsy is taken from bone marrow and the biopsy is washed once in cell growth medium. The growth medium contains HAM F12 and 15 millimolar HEPES buffer, 70 micromole/liter gentomycin sulfate, 2.2 micromole/liter amphotericin, 0.3 millimole/liter ascorbic acid, and 20% fetal calf serum. Specific growth factor(s) are included in the growth medium for the induction of specific cell lineage. For example transforming growth factor-beta is included in the medium for the induction of chondrocyte differentiation whereas fibroblast growth factor is included in the medium for the induction of tenocyte differentiation. The stem cells are cultured in the medium and counted to determine viability. The differentiated cells are grown in minimal essential culture medium containing HAM F12 and 15 millimolar HEPES buffer and 5 to 7.5% autologous serum in a CO₂ incubator at 37 degrees Celsius and handled in a Class 100 laboratory. Other compositions of culture medium may be used for culturing the cells. The cells are trypsinized using trypsin EDTA for 5 to 10 minutes and counted using Trypan Blue viability staining in a Buurker-Turk chamber. The cell count is adjusted to 7.5×10⁵ cells per milliliter.

A type I/III collagen membrane from Geistlich Sohne (Switzerland) or Matricel GmbH (Kaiserstr., Germany) is used as a support matrix. The matrix is cut to a suitable size to fit the bottom of the well in the NUNCLON^{™} Delta 6-well tissue culture tray and placed in the well under aseptic conditions (NUNC (InterMed) Roskilde, Denmark). A small amount of the cell culture medium containing serum is applied to the matrix to be absorbed into the matrix and to keep the matrix wet at the bottom of the well.

Approximately 10⁶ cells in 1 milliliter of culture medium are placed directly on top of the matrix, dispersed over the surface of the matrix. The tissue culture plate is then incubated in a CO₂ incubator at 37 degrees Celsius for 60 minutes. From 2 to 5 milliliters of tissue culture medium containing 5 to 7.5% serum is carefully added to the tissue culture well containing the cells. The pH is adjusted to about 7.4 to 7.5 if necessary. The plate is incubated for 3 to 7 days with a medium change at day 3.

At the end of the incubation period the medium is decanted and the cell-seeded support matrix is washed. The support matrix is then implanted, cell-side down, into the defect site, and optionally is covered with a covering patch. The defect is then permitted to heal on its own.

## Claims

1. A tissue repair composition for use in the treatment of a tissue defect or to regenerate a tissue *in vivo,* comprising
a) a collagen membrane and;
b) cultured chondrocytes adhered to a surface of the membrane,
wherein the membrane comprises a combination of collagen type I and collagen type III;
and wherein the chondrocytes are brought into contact with one or more portions of the membrane such that a portion of the chondrocytes migrate into a surface of the membrane up to a predetermined maximum depth of 50% of the membrane during seeding; and
wherein when the composition is implanted, the chondrocytes migrate to and populate a site of transplantation.

2. The tissue repair composition for use according to claim 1 wherein the collagen membrane is cell free prior to contact with the cultured chondrocytes.

3. The tissue repair composition for use according to any one of the preceding claims wherein the collagen membrane is resorbable.

4. The tissue repair composition for use according to any one of the preceding claims wherein the collagen membrane is porcine.

5. The tissue repair composition for use according to any one of the preceding claims wherein the structure is implantable.

6. The tissue repair composition for use according to any one of the preceding claims wherein the tissue defect is a cartilage defect of the knee.

7. The tissue repair composition for use according to claim 6, wherein the cartilage defect is an articular cartilage defect.

8. The tissue repair composition for use according to any one of the preceding claims wherein the cultured chondrocytes are obtained by *in vitro* culturing of a cartilage biopsy.

9. The tissue repair composition for use according to any of the preceding claims wherein the maximum depth is 25 percent of the depth of the membrane.

10. The tissue repair composition for use according to any of the preceding claims wherein the maximum depth is 10 percent of the depth of the membrane.

11. The tissue repair composition for use according to any of the preceding claims wherein the membrane comprises autologous material.

12. The tissue repair composition for use according to any of the preceding claims wherein the membrane comprises allogeneic material.

## Patentansprüche

1. Gewebereparaturzusammensetzung zur Verwendung bei der Behandlung eines Gewebedefekts oder zum Regenerieren eines Gewebes *in vivo,* umfassend
a) eine Kollagenmembran und;
b) kultivierte Chondrozyten, die an einer Oberfläche der Membran haften,
wobei die Membran eine Kombination aus Kollagen Typ I und Kollagen Typ III umfasst;
und wobei die Chondrozyten mit einem oder mehreren Teilen der Membran in Kontakt gebracht werden, so dass während des Ansäens ein Teil der Chondrozyten bis zu einer vorbestimmten maximalen Tiefe von 50 % der Membran in eine Oberfläche der Membran wandert; und wobei, wenn die Zusammensetzung implantiert wird, die Chondrozyten zu einer Transplantationsstelle wandern und diese besiedeln.

2. Gewebereparaturzusammensetzung zur Verwendung nach Anspruch 1, wobei die Kollagenmembran vor dem Kontakt mit den kultivierten Chondrozyten zellfrei ist.

3. Gewebereparaturzusammensetzung zur Verwendung nach einem der vorhergehenden Ansprüche, wobei die Kollagenmembran resorbierbar ist.

4. Gewebereparaturzusammensetzung zur Verwendung nach einem der vorhergehenden Ansprüche, wobei die Kollagenmembran porciner Herkunft ist.

5. Gewebereparaturzusammensetzung zur Verwendung nach einem der vorhergehenden Ansprüche, wobei die Struktur implantierbar ist.

6. Gewebereparaturzusammensetzung zur Verwendung nach einem der vorhergehenden Ansprüche, wobei der Gewebedefekt ein Knorpeldefekt des Knies ist.

7. Gewebereparaturzusammensetzung zur Verwendung nach Anspruch 6, wobei der Knorpeldefekt ein Gelenkknorpeldefekt ist.

8. Gewebereparaturzusammensetzung zur Verwendung nach einem der vorhergehenden Ansprüche, wobei die kultivierten Chondrozyten durch In-vitro-Kultivierung einer Knorpelbiopsie erhalten werden.

9. Gewebereparaturzusammensetzung zur Verwendung nach einem der vorhergehenden Ansprüche, wobei die maximale Tiefe 25 Prozent der Tiefe der Membran beträgt.

10. Gewebereparaturzusammensetzung zur Verwendung nach einem der vorhergehenden Ansprüche, wobei die maximale Tiefe 10 Prozent der Tiefe der Membran beträgt.

11. Gewebereparaturzusammensetzung zur Verwendung nach einem der vorhergehenden Ansprüche, wobei die Membran autologes Material umfasst.

12. Gewebereparaturzusammensetzung zur Verwendung nach einem der vorhergehenden Ansprüche, wobei die Membran allogenes Material umfasst.

## Revendications

1. Composition de réparation tissulaire, pour une utilisation destinée au traitement d'un défaut tissulaire ou afin de régénérer un tissu *in vivo,* comprenant :
a) une membrane de collagène ; et
b) des chondrocytes cultivés adhérant à une surface de la membrane ;
dans laquelle la membrane comprend une combinaison de collagène de type I et de collagène de type III ; et où les chondrocytes sont mis en contact avec une ou plusieurs portions de la membrane de façon à ce qu'une portion des chondrocytes migre dans une surface de la membrane jusqu'à une profondeur maximale prédéterminée de 50% de la membrane lors de l'ensemencement ; et
où, lorsque la composition est implantée, les chondrocytes migrent vers un site de transplantation et le peuplent.

2. Composition de réparation tissulaire pour une utilisation selon la revendication 1, dans laquelle la membrane de collagène est dépourvue de cellules préalablement au contact avec les chondrocytes cultivés.

3. Composition de réparation tissulaire pour une utilisation selon l'une quelconque des revendications précédentes, dans laquelle la membrane de collagène est résorbable.

4. Composition de réparation tissulaire pour une utilisation selon l'une quelconque des revendications précédentes, dans laquelle la membrane de collagène est porcine.

5. Composition de réparation tissulaire pour une utilisation selon l'une quelconque des revendications précédentes, dans laquelle la structure est implantable.

6. Composition de réparation tissulaire pour une utilisation selon l'une quelconque des revendications précédentes, dans laquelle le défaut tissulaire est un défaut de cartilage du genou.

7. Composition de réparation tissulaire pour une utilisation selon la revendication 6, dans laquelle le défaut de cartilage est un défaut de cartilage articulaire.

8. Composition de réparation tissulaire pour une utilisation selon l'une quelconque des revendications précédentes, dans laquelle les chondrocytes cultivés sont obtenus par la culture *in vitro* d'une biopsie de cartilage.

9. Composition de réparation tissulaire pour une utilisation selon l'une quelconque des revendications précédentes, dans laquelle la profondeur maximale est de 25% de la profondeur de la membrane.

10. Composition de réparation tissulaire pour une utilisation selon l'une quelconque des revendications précédentes, dans laquelle la profondeur maximale est de 10% de la profondeur de la membrane.

11. Composition de réparation tissulaire pour une utilisation selon l'une quelconque des revendications précédentes, dans laquelle la membrane comprend un matériau autologue.

12. Composition de réparation tissulaire pour une utilisation selon l'une quelconque des revendications précédentes, dans laquelle la membrane comprend un matériau allogène.
